# EUROPEAN PATENT APPLICATION

(11) **EP 4 407 351 A1**
(43) Date of publication of application: **31.07.2024**
(21) Application number: 23153856.2
(22) Date of filing: 30.01.2023
(51) Int. Cl.: G01T 1/20

(54) **PIXELATED SCINTILLATOR**

(71) Applicant: Koninklijke Philips N.V., 5656 AG Eindhoven (NL)
(72) Inventor: JACOBS, Johannes, Eindhoven (NL); SIMON, Matthias, Eindhoven (NL)
(74) Representative: Philips Intellectual Property & Standards

(57) **Abstract**

The present invention relates to a method of manufacturing a pixelated scintillator. According to the method, first laser radiation is irradiated to a plurality of first regions of a substrate according to a predefined geometry of the pixelated scintillator to cause a structural modification of a material in the plurality of first regions of the substrate such that wet chemical etching occurs at a higher rate at the plurality of irradiated first regions than at a non-irradiated region. Wet chemical etching is performed to form a plurality of first openings having the predefined geometry of the pixelated scintillator. The plurality of first openings are filled with at least one scintillator material to form the pixelated scintillator. The resulting pixelated scintillator embedded in the substrate may be bonded to a corresponding photosensitive read-out sensor substrate. This may enable realization of cost-effective, parallax-free, single- or multi-layer radiation detectors with enhanced spatial resolution and sensitivity.

## Description

### FIELD OF THE INVENTION

The present invention relates to a pixelated scintillator, and in particular to a method of manufacturing a pixelated scintillator, to a pixelated scintillator obtainable by the method, and to a radiation detector.

### BACKGROUND OF THE INVENTION

Current radiation detectors used in medical imaging mostly comprise a non-pixelated micro-columnar scintillator layer bonded to a read-out sensor substrate which is based on a large array of small, electronically-addressable, photosensitive pixels. An inherent problem of these so-called indirect conversion radiation detectors may be the reduced lateral resolution due to scintillation light blurring in the scintillator layer. In addition, the spatial resolution of such detectors may deteriorate from the centre towards the edge of the detector due to an increase of parallax errors. These effects may become even more pronounced as the pixel sizes of X-ray detectors are reduced and/or of the X-ray detectors include multiple scintillator layers.

The above problems may, at least partly, be solved by replacing the non-pixelated scintillator by a pixelated scintillator, i.e., an array of scintillator elements, also referred to as voxels, embedded in a substrate, or deposited on top of a substrate. Various methods to realize a radiation detector based on a pixelated scintillator have been proposed. However, most methods may provide only limited detector performance gain and/or suffer from high manufacturing complexity.

### SUMMARY OF THE INVENTION

There may be a need to provide an improved method of manufacturing a pixelated scintillator for a radiation detector, such as X-ray detector or gamma-ray detector.

The object of the present invention is solved by the subject-matter of the independent claims, wherein further embodiments are incorporated in the dependent claims. It should be noted that the following described aspects of the invention apply also for the method of manufacturing a pixelated scintillator, the pixelated scintillator obtainable by the method, and the radiation detector.

According to a first aspect of the present invention, there is provided a method of manufacturing a pixelated scintillator, the method comprising:
- irradiating, with first laser radiation, a plurality of first regions of a substrate according to a predefined geometry of the pixelated scintillator to cause a structural modification of a material in the plurality of first regions of the substrate such that wet chemical etching occurs at a higher rate at the plurality of irradiated first regions than at a non-irradiated region;
- performing wet chemical etching to form a plurality of first openings having the predefined geometry of the pixelated scintillator; and
- filling the plurality of first openings with at least one scintillator material to form the pixelated scintillator.

The present disclosure proposes a new method to manufacture a pixelated scintillator, e.g., a focused pixelated scintillator. Laser microfabrication, e.g., femto-laser microfabrication, followed by wet etching is used to create a plurality of first openings in a substrate, e.g., glass or plastic substrate. The openings may be arranged in the form of an array. The large flexibility of laser beam writing may make it possible to produce openings with a customized geometry, e.g. high aspect ratio openings, openings with varying height, wall thickness, aspect ratio, shape, pitch, angular obliqueness, etc., across the substrate. Hereafter, these openings are filled with one or more scintillator materials, for example by means of screen printing or binder jetting. The filled openings may be referred to as scintillator elements, or voxels, of the pixelated scintillator. Thus, the openings are filled in order to define scintillator elements, or voxels, of the pixelated scintillator. The customized geometry of the openings therefore facilitates the provision of scintillator elements that also have a customized geometry. The subsequent process of filling of the openings may enable the provision of a pixelated scintillator from a selection of scintillator materials, and scintillator materials in different forms. For instance, powder-based scintillator materials may be used. The process of filling the openings may also enable the pixelated scintillator using fabrication methods with modest costs, and a pixelated scintillator which is embedded, and therefore protected, in a solid large-area substrate.

The resulting pixelated scintillator may be bonded to a corresponding photosensitive read-out sensor substrate to realize a single- or multi-layer radiation detectors with enhanced spatial resolution and sensitivity.

The proposed fabrication method of the pixelated scintillator may address increased demands on quality, area uniformity, robustness and/or cost reductions, which can consequently facilitate new medical imaging methods, such as spectral X-ray imaging and phase-contrast imaging.

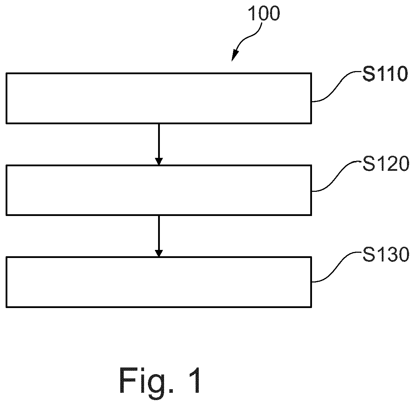

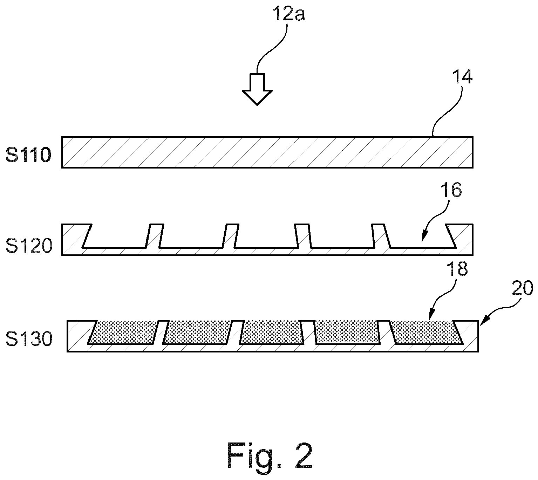

This will be explained in detail hereinafter and in particular with respect to the examples shown in Figs. 1 and 2.

According to an embodiment of the present invention, prior to the step of filling the plurality of first openings with at least one scintillator material to form the pixelated scintillator, the method further comprises coating at least part of an interior surface of at least one first opening with an optically-reflecting material or a non-transparent material.

*In some examples, the side walls of the first openings are coated with an optically reflecting material or a non-transparent material. In this way, the optical signal is confined within each scintillator element. This reduces the ability of optical signals to escape to neighbouring photosensitive pixels on the read-out sensor.*

*In some examples, a bottom surface of the first openings is coated with an optically-reflecting material. Including a coated layer with an optically reflecting material to the bottom of scintillator elements in glass substrate may increase scintillation light output of the pixelated scintillator.*

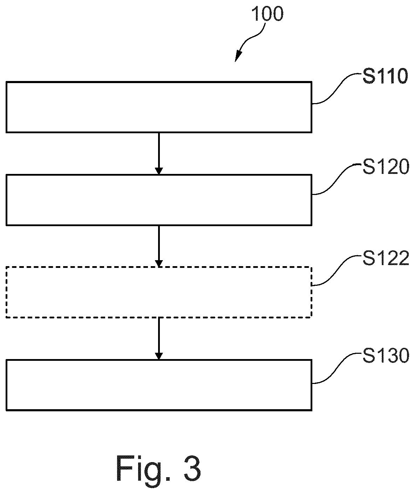

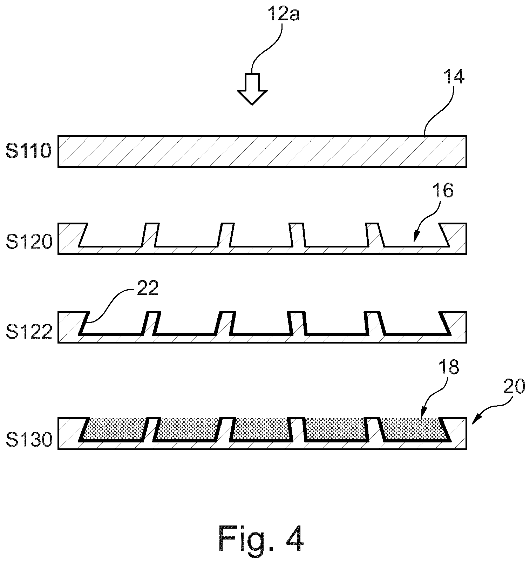

*In some examples, both the side walls and the bottom surface of the first openings are coated with an optically-reflecting material or a non-transparent material. This will be explained in detail hereinafter and in particular with respect to the example shown in* *Figs. 3* *and* *4**.*

According to an embodiment of the present invention, after the step of filling the plurality of first openings with at least one scintillator material to form the pixelated scintillator, the method further comprises coating at least one filled first opening with an optically-reflecting material or a non-transparent material.

In other words, one or more filled openings, also known as scintillator *elements,* or voxels, may be coated with an optically reflecting material or a non-transparent material. This may be used to fabricate a pixelated scintillator with a so-called "front-irradiation" geometry.

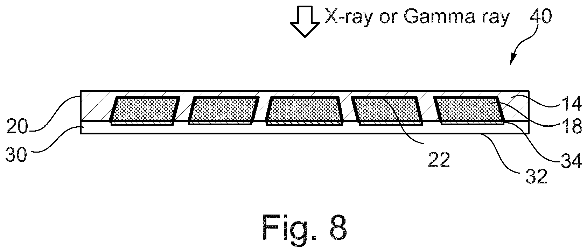

This will be explained hereinafter and in particular with respect to the example shown in Fig. 8.
According to an embodiment of the present invention, the method further comprises:
- irradiating, with second laser radiation, one or more second regions of the substrate,
- performing wet etching to form one or more second openings, and
- filling the one or more second openings with an optically reflecting material or a non-transparent material, wherein the one or more second regions are different from the plurality of first regions, and at least one second region is arranged between two adjacent first regions.

These additional steps may allow to manufacture a pixelated scintillator with reflecting or non-transparent walls. First, high-aspect-ratio trenches, such as high-aspect-ratio angulated trenches, are fabricated in a substrate (e.g., glass substrate) using a laser exposure (e.g., femto-laser exposure) followed by wet etching. A thin solid layer of glass remains as supporting base substrate for the system of trenches. Second, the trenches may be filled with an optically reflecting material. For example, a reflecting TiO₂ pigment layer may be applied by capillary filling from a TiO₂-based paint. Alternatively, trenches that are formed by the etching step may be filled with a non-transparent trench walls.

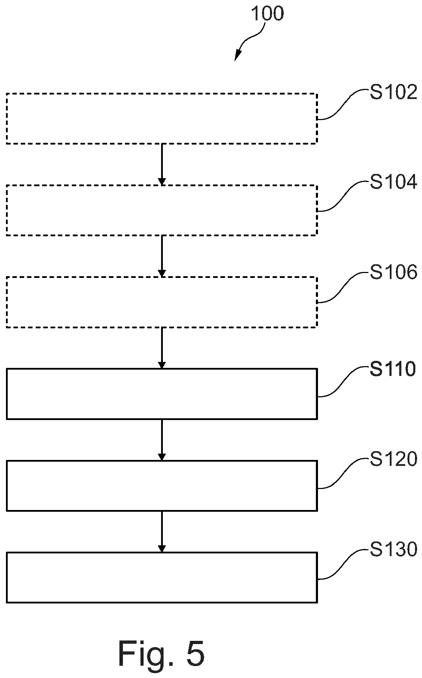

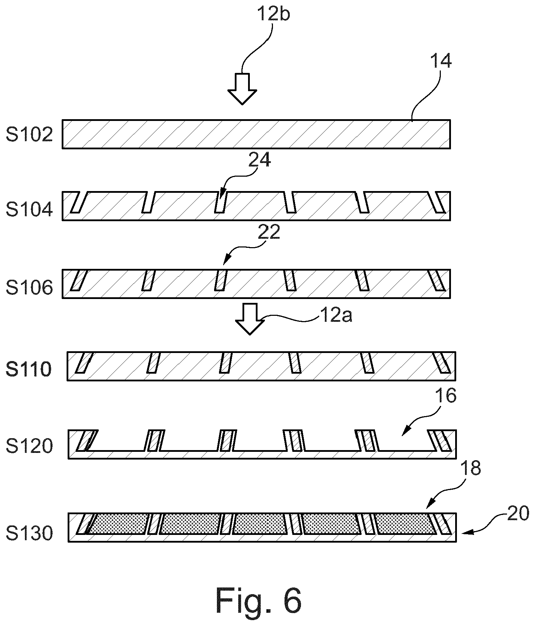

This will be explained hereinafter and in particular with respect to the example shown in Figs. 5 and 6.

According to an embodiment of the present invention, the method further comprises bending the substrate in one or two dimensions to shape the substrate according to a desired curvature.

In this embodiment, a bendable substrate for scintillator (e.g., thin glass or plastic foil) may be used to enable fabrication of a curved radiation detector.

According to an embodiment of the present invention, at least one of the first laser radiation and the second laser radiation is produced by a focused pulsed femtosecond laser.

Femto-laser micromachining technology has demonstrated the manufacturability of various monolithically integrated devices such as optofluidic, optomechanical and photonic devices. As glasses (such as but not limited to fused silica), polymers, dielectrics or crystals are usually used as transparent substrate materials, laser beams can be focused almost anywhere inside the substrate material and the energy can be deposited almost anywhere in the volume. The energy deposition causes a structural modification of the material, so that subsequent wet chemical etching occurs at a much higher rate at exposed areas than at non-exposed areas. This localized enhanced susceptibility to wet chemical etching (the etch selectivity) depends on various femto-laser parameters, such as pulse duration, pulse energy, and pulse repetition rate.

According to an embodiment of the present invention, the plurality of first openings are angulated towards a common focal point.

The resulting pixelated scintillator may have scintillator element sidewalls which are better aligned towards the X-ray or gamma-ray focal spot, thereby reducing pixel edge effects. Examples of the angulated pixelated scintillators are shown in Figs. 2, 4 and 6.

According to an embodiment of the present invention, the at least one scintillator material filled in the plurality of first openings has a relatively greater thickness in a centre of the substrate than at an edge of the substrate.

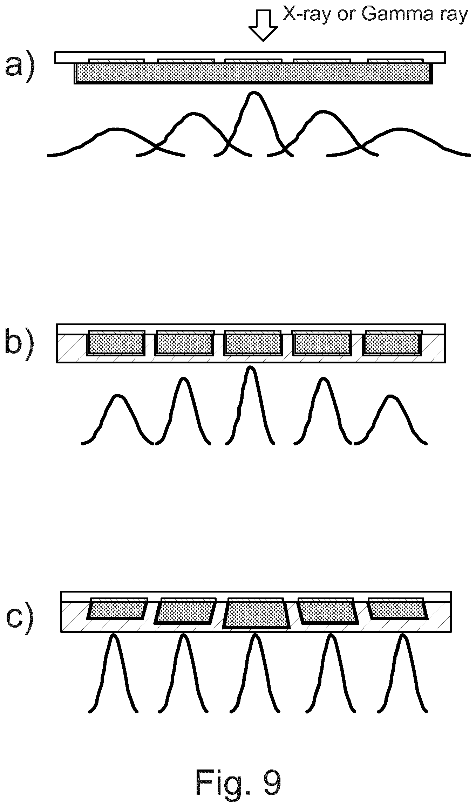

In this way, a uniform scintillator absorption depth across the whole detector can be realized. *This will be discussed in detail hereinafter and in particular with respect to the examples shown* in Figs. 9(a)-9(c).

According to an embodiment of the present invention, at least two scintillator materials with different scintillator light emission spectra are filled in the plurality of first openings.

The sensitivity of X-ray detectors that employ pixelated scintillators may be limited by an improper match between spectral properties of scintillator and photodiode. By using multiple scintillator materials, it becomes possible to match the emission spectrum of the scintillator materials in the scintillator elements with the photodiode sensitivity spectrum.

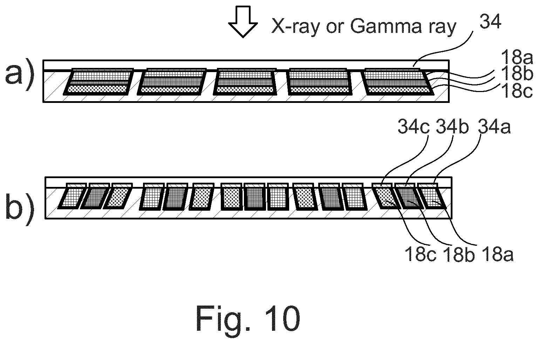

This will be explained in detail hereinafter and in particular with respect to the examples shown in Figs. 10(a) and 10(b).

According to an embodiment of the present invention, the at least two scintillator materials are deposited in at least one of the first openings consecutively. Alternatively or additionally, at least one scintillator element of the pixelated scintillator comprises a plurality of sub- scintillator elements, and at least two sub-scintillator elements of the at least one scintillator element are filled with different scintillator materials.

This will be explained in detail hereinafter and in particular with respect to the examples shown in Figs. 10(a) and 10(b).

According to an embodiment of the present invention, the substrate comprises a glass material or a plastic material.

According to a second aspect of the present invention, there is provided a pixelated scintillator obtainable by a method according to the first aspect and any associated example.

According to a third aspect of the present invention, there is provided a radiation detector that comprises a pixelated scintillator according to the second aspect and a pixelated and photosensitive read-out sensor.

Optical crosstalk is caused by the a photosensitive pixel on the sensor receiving optical signals from multiple scintillator elements. The radiation detector as described herein may reduce this effect by providing improved lateral alignment between photosensitive pixel and scintillator element and minimizing a distance (gap) between the scintillator and the sensor.

In addition, the sensitivity of X-ray detectors that employ pixelated scintillators may be limited by a low fill factor. The fill factor of the radiation detection as described herein may be improved by providing accurate alignment of scintillator elements with the photosensitive area of pixels on the sensor.

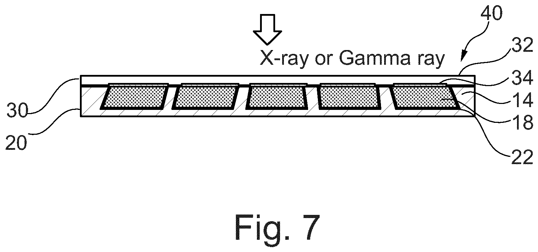

This will be explained in detail hereinafter and in particular with respect to the example shown in Fig. 7.

According to an embodiment of the present invention, the radiation detector is a multi-layer detector comprising a plurality of stacked pixelated scintillators.

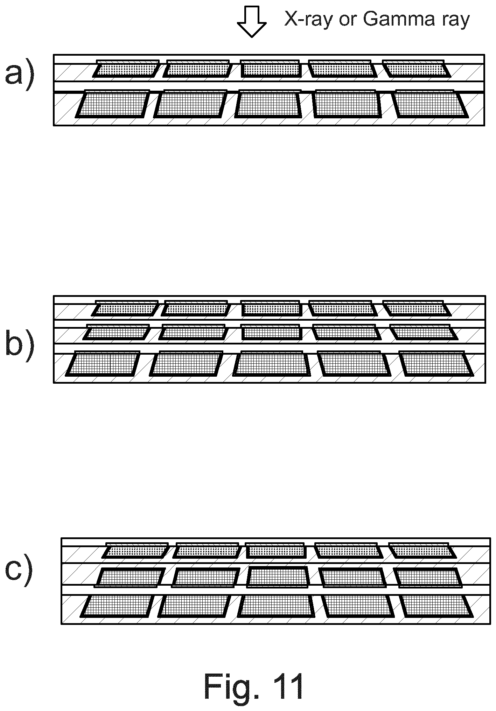

The resulting pixelated focused scintillators may be advantageous for multi-layer detectors since it reduces the cross-contamination of signals between different detector layers. This will be explained hereinafter and in particular with respect to the example shown in Figs. 11(a)-11(c).

According to an embodiment of the present invention, the pixelated scintillator and the pixelated and photosensitive read-out sensor are configured and arranged to form the radiation detector in a so-called "back-irradiation" geometry or in a so-called "front-irradiation" geometry.

A pixelated scintillator may be integrated into an X-ray detector in either of these geometries. In the front-irradiation geometry, X-rays or gamma rays are firstly incident on the scintillator, where they are absorbed. Their resulting scintillation light is then detected by the photosensitive read-out sensor. This is the most common geometry in current X-ray detectors. In a back-irradiation geometry, X-rays or gamma rays are firstly incident on the photosensitive read-out sensor substrate. The X-rays or gamma rays pass through the photosensitive read-out sensor substrate and are then absorbed by the scintillator. Their resulting scintillation light is then detected by the photosensitive read-out sensor.

An example of a radiation detector in the back-irradiation geometry is shown in Fig. 7. An example of a radiation detector in the front-irradiation geometry is shown in Fig. 8.

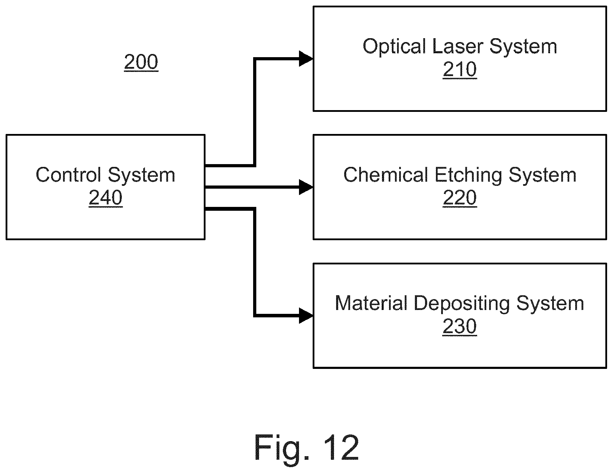

The pixelated scintillator may be fabricated using a manufacturing system comprising an optical laser system, a chemical etching system, and a material deposition system, and a control system comprising one or more controllers. The control system may be configured to control the optical laser system to irradiate, with first laser radiation, a plurality of first regions of a substrate according to a predefined geometry of the pixelated scintillator to cause a structural modification of a material in the plurality of first regions of the substrate such that wet chemical etching occurs at a higher rate at the plurality of irradiated first regions than at a non-irradiated region. The control system may be configured to control the chemical etching system to perform wet chemical etching to form a plurality of first openings having the predefined geometry of the pixelated scintillator. The control system may be configured to control the material depositing system to fill the plurality of first openings with at least one scintillator material to form the scintillator elements of the pixelated scintillator. This will be explained in detail hereinafter and in particular with respect to the example shown in Fig. 12.

A computer program may be provided comprising instructions to cause the manufacturing system to execute the steps of the method according to the first aspect and any associated example.

A computer-readable medium may be provided having stored thereon the computer program.

### BRIEF DESCRIPTION OF THE DRAWINGS

These and other aspects of the invention will be apparent from and elucidated further with reference to the embodiments described by way of examples in the following description and with reference to the accompanying drawings, in which
Fig. 1 illustrates a flowchart describing an example of a method of manufacturing a pixelated scintillator.
Fig. 2 illustrates an example of a manufacturing process in connection with Fig. 1.
Fig. 3 illustrates a flowchart describing another example of a method of manufacturing a pixelated scintillator.
Fig. 4 illustrates another example of a manufacturing process in connection with Fig. 3.
Fig. 5 illustrates a flowchart describing a further example of a method of manufacturing a pixelated scintillator.
Fig. 6 illustrates another example of a manufacturing process in connection with Fig. 5.
Fig. 7 schematically shows an example of a radiation detector fabricated by coupling the pixelated scintillator to a read-out sensor in a back-irradiation geometry.
Fig. 8 schematically shows an example of a radiation detector fabricated by coupling the pixelated scintillator to a read-out sensor in a front-irradiation geometry.
Fig. 9(a)-9(c) illustrate schematically how the spatial resolution of a radiation detector in back-irradiation geometry can be improved by optimizing the design of a scintillator coupled to the read-out sensor.
Figs. 10(a) and 10(b) show examples in which scintillator elements composed of multiple scintillator materials can be used to improve detective quantum efficiency (DQE) for specific clinical applications.
Figs. 11(a)-11(c) illustrate that pixelated focused scintillators are advantageous for multi-layer detectors to minimize cross contamination of signals between different detector layers.
Fig. 12 illustrates an example of a system for manufacturing a pixelated scintillator.

It should be noted that the figures are purely diagrammatic and not drawn to scale. In the figures, elements which correspond to elements already described may have the same reference numerals. Examples, embodiments or optional features are not to be understood as limiting the invention as claimed.

### DETAILED DESCRIPTION OF EMBODIMENTS

The present invention will now be described more fully hereinafter with reference to the accompanying drawings, in which preferred embodiments of the invention are shown. This invention may, however, be embodied in many different forms and should not be construed as limited to the embodiments set forth herein. Rather, these embodiments are provided so that this disclosure will be thorough and complete, and will convey the scope of the invention to those skilled in the art. Those of ordinary skill in the art realize that the following descriptions of the embodiments of the present invention are illustrative and are not intended to be limiting in any way. Other embodiments of the present invention will readily suggest themselves to such skilled persons having the benefit of this disclosure. Like numbers refer to like elements throughout.

Although the following detailed description contains many specifics for the purposes of illustration, anyone of ordinary skill in the art will appreciate that many variations and alterations to the following details are within the scope of the invention. Accordingly, the following embodiments of the invention are set forth without any loss of generality to, and without imposing limitations upon, the claimed invention.

Various methods to manufacture pixelated scintillators have been proposed in the past, but mostly they suffer from one or more drawbacks, including high costs. For example, dry reactive ion etching of silicon wafers followed by filling with CsI(Tl) in a melting process may be not suitable to produce focused trenches in silicon because etching process is very sensitive to the silicon crystal orientation. Laser machining (ablation) of CsI(Tl) films followed by deposition of reflective coatings in the laser-cut trenches may be used to improve optical isolation between scintillator elements. However, this method may suffer from significant reduction of scintillator sensitivity, which can probably be attributed to laser-induced microstructural changes in the CsI(Tl) film. Additive manufacturing of scintillator elements, for example by 3D printing (e.g., photopolymerisation) from a scintillator particle-in-binder powder system, may be used to produce 3D-printed scintillator structures. However, such structures may be mechanically not robust enough to survive a subsequent assembly process to fabricate an X-ray detector. The choice of scintillators may also be limited by the ability to make a printable paste.

In order to address one or more of the above-described problems, the present disclosure proposes a method of manufacturing a pixelated scintillator, which may enable a selection from a large variety of low-cost scintillators materials (e.g., powder-based scintillator materials) and fabrication methods to realize a pixelated scintillator which is embedded and protected in a solid large-area substrate.

Fig. 1 illustrates a flowchart describing a method 100 for manufacturing a pixelated scintillator, i.e., an array of scintillator elements. The method will be described in connection with Fig. 2.

In step S 110, first laser radiation 12a is irradiated to a plurality of first regions of a substrate 14 according to a predefined geometry of the pixelated scintillator to cause a structural modification of a material in the plurality of first regions of the substrate such that wet chemical etching occurs at a higher rate at the plurality of irradiated first regions than at a non-irradiated region.

The substrate may be made of a material that is transparent or at least partially transparent to the first laser radiation. For example, glasses (such as but not limited to fused silica), polymers, dielectrics, or crystals may be used as transparent substrate materials, because laser beams can be focused almost anywhere inside the material and the energy can be deposited almost anywhere in the volume.

Thanks to the non-linear nature of the ultrafast laser-matter interaction, the relatively low laser energy is locally absorbed wherever the laser spot is focused. The non-linear absorption of laser energy makes it possible to use a moderate average laser power, although enormous instantaneous powers are locally reached during the laser exposure. It is even possible to fabricate features smaller than the wavelength of the laser itself (e.g., 1030 nm). As an example, the first laser radiation may be produced by a focused pulsed femtosecond laser. The "femtoprint" process using the femto-laser micromachining technology, which is a subtractive 3D printing technique, may be able to create narrow deep channels in glass with high trench aspect ratios (TAR > 100), which are comparable to or higher than obtained with competitive processes like Reactive Ion Etching (RIE).

The energy deposition causes a structural modification of the material, so that subsequent wet chemical etching occurs at a high much higher rate at exposed region(s) than at non-exposed region(s). This localized enhanced susceptibility to wet chemical etching (the etch selectivity) depends on various laser parameters, such as pulse duration, pulse energy and repetition rate.

Examples of the predefined geometry of the pixelated scintillator include, inter alia, a one-dimensional (1D) design, two-dimensional (2D) design (e.g., rectangular or hexagonal scintillator elements), and customized scintillator element design (e.g.,1½D) with flexible scintillator element pitch, scintillator element ratio and/or scintillator element geometry. The predefined geometry of the pixelated scintillator may include information like height, wall thickness, aspect ratio, scintillator element shape, scintillator element pitch, angular obliqueness, etc., across the substrate.

In step S120, wet chemical etching is performed to form a plurality of first openings having the predefined geometry of the pixelated scintillator.

As shown in Fig. 2, laser exposure may be controlled in such a way that the first openings do not extend completely to the other side of the substrate 14. A thin layer of the substrate remains as supporting base substrate for the scintillator elements of the pixelated scintillator.

In some examples, as shown in Fig. 2, the optical laser system that generates the first laser radiation may be designed and programmed to write a specified structure (e.g., array) of the first openings 16 characterized by a small continuous increase of trench angles from the centre towards the periphery of the substrate 14. The optical laser system may also take into account of the difference of refractive indexes at the air-substrate interface and the increased optical path lengths for the first openings 16 towards the periphery of the substrate 14.

In step S130, the plurality of first openings 16 is filled with at least one scintillator material 18 to form the pixelated scintillator 20.

The plurality of first openings 16 may be filled with one or more selected scintillator material using any suitable application method. Depending on the detector design and its imaging applications, a large variety of scintillator materials, e.g., GOS, CsI, perovskites nanoparticles, quantum dot materials, and so forth, may be selected with different additives, such as dopants, binders, etc., and in different formats including, but not limited to, powders, pastes, and suspensions. Also, there is a large choice in possible scintillator deposition methods including, but not limited to, 3D printing, binder jetting, screen printing, slot die coating, flex printing, spin coating, powder melting, and the like.

The method as described herein may enable the selection from a variety of cheap (e.g., powder-based) scintillators materials and fabrication methods to realize a pixelated scintillator which is embedded and protected in a solid large-area substrate. Furthermore, the method as described herein may allow to provide improved environmental and mechanical protection by embedding small vulnerable scintillator structures in a solid substrate, e.g., glass substrate. The fill factor may be improved by accurate alignment of scintillator elements with the active photosensitive area of pixels on the sensor. The method may enable to manufacture focused scintillator elements in a predefined geometrical shape and sharp boundaries. For example, the focused scintillator, i.e., angulated scintillator, shown in Fig. 2 may reduce pixel edge effects. The method as described herein may reduce optical crosstalk in an X-ray detector by realizing lateral alignment between photosensitive pixel and scintillator element and minimizing the distance (gap) between sensor and scintillator. This will be described hereinafter and in particular with respect to the example shown in Fig. 7.

The pixelated scintillator may comprise a one-dimensional (1D), two-dimensional (2D), or three-dimensional (3D) array of scintillator elements. The pixelated scintillator may be used in radiation imaging detectors in order to detect and thereby image ionizing radiation such as X-ray and gamma radiation. The pixelated scintillator may be used for example in medical imaging systems such as positron emission tomography (PET), single photon emission computed tomography (SPECT), and computed tomography (CT) imaging systems. The pixelated scintillator may be used in non-medical imaging systems, such as industrial radiography for inspection of industrial parts. The materials of such scintillator elements are selected so as to generate a pulse of scintillation light in response to each received X-ray or gamma quant. The scintillation light is subsequently detected by a photodetector array that is optically coupled to the scintillator elements.

The point spread function of current radiation detectors which are mostly based on continuous (non-pixelated) scintillator may be broadened by blurring of scintillation light in the scintillator after interaction of X-rays or gamma rays with the scintillator. This is mainly caused by scintillator light scattering and reflection effects at scintillator crystal grain boundaries. These effects are most pronounced in polycrystalline (powder) layers, such as GOS, but also occur in columnar structured layers, such as CsI. To that end, it is an option for the pixelated scintillator of the present disclosure to be configured to have an optical separation layer between the scintillator elements to provide the optical separation of the scintillator such that the scintillation photons generated in a scintillator element cannot easily pass out of that scintillator element and into to another scintillator element. When scintillator elements are optically separated, the majority of the generated scintillation photons are contained in the scintillator element where they were generated, for example by means of total internal reflection. Examples of the separator material may include, but are not limited to, an optically reflecting material and a non-transparent material. In this way, the optical signal is, independently of the scintillator material type, confined within each scintillator element. Escape of optical signals to neighbouring photosensitive pixels on the read-out sensor is nearly impossible.

There are various approaches to manufacture a pixelated scintillator with an optical separation layer.
Fig. 3 illustrates an exemplary flowchart describing a method 100 for manufacturing a pixelated scintillator with an optical separation layer. The method will be described in connection with Fig. 4.

In step S 110, first laser radiation 12a is irradiated to a plurality of first regions of a substrate 14 according to a predefined geometry of the pixelated scintillator to cause a structural modification of a material in the plurality of first regions of the substrate such that wet chemical etching occurs at a higher rate at the plurality of irradiated first regions than at a non-irradiated region.

In step S120, wet chemical etching is performed to form a plurality of first openings 16 having the predefined geometry of the pixelated scintillator.

In step S 122, at least part of an interior surface of at least one first opening 16 is coated with a separator material 22, such as an optically reflecting material or a non-transparent material. In some examples, as shown in Fig. 4, both the side walls and the bottom surface of the first openings are coated with the separator material. In some examples (not shown), only side walls of the first openings 16 are coated with the separator material. There is a large choice in possible material coating methods for the separator material. For example, atomic layer deposition (ALD), which is a well-established technique, may be used to deposit highly conformal multi-layer coatings (e.g., SiO₂/Al₂O₃/Al) into high-aspect ratio trenches. Other material coating techniques may be based on thermal evaporation, thermal melting, capillary filling from suspension, or paste.

In step S130, the plurality of first openings 16 are filled with at least one scintillator material 18 to form the pixelated scintillator 20.

Fig. 5 illustrates an exemplary flowchart describing a method 100 for manufacturing a pixelated scintillator with reflecting or non-transparent trench walls. The method will be described in connection with Fig. 6.

In step S 102, a second laser radiation 12b is irradiated to one or more second regions of the substrate 14. The first laser radiation 12a and the second laser radiation 12b may represent different sequences of the laser exposure.

In step S104, wet etching is performed to form one or more second openings 24. Similarly, the laser exposure may be controlled in such a way that the second openings 24 do not extend completely until the other side of the substrate 14. A thin solid layer of substrate remains as supporting base substrate for the system of the second openings 24.

In step S 106, the one or more second openings 24 are filled with a separator material 22, such as an optically reflecting material or a non-transparent material. For example, a reflecting TiO₂ pigment layer may be applied by capillary filling from a TiO₂-based paint. The one or more second regions are different from the plurality of first regions, and at least one second region is arranged between two adjacent first regions.

In step S 110, first laser radiation 12a is irradiated to a plurality of first regions of a substrate 14 according to a predefined geometry of the pixelated scintillator to cause a structural modification of a material in the plurality of first regions of the substrate such that wet chemical etching occurs at a higher rate at the plurality of irradiated first regions than at a non-irradiated region.

In step S120, wet chemical etching is performed to form a plurality of first openings 16 having the predefined geometry of the pixelated scintillator.

In step S130, the plurality of first openings 16 is filled with at least one scintillator material 18 to form the pixelated scintillator 20.

In the example shown in Figs. 5 and 6, the reflecting or non-transparent trench walls are formed prior to the scintillator elements. In some other examples (not shown), the separator layer may be formed after the scintillator elements. In other words, steps S 102 to S 106 may be performed after steps S110 to S130.

In some examples, a protective layer may be added on the scintillator substrate of the pixelated scintillator, e.g., the pixelated scintillator 20 shown in Figs. 2, 4 and 6, to prevent loss of scintillator material from the scintillator elements and/or to prevent loss of separator material from trenches.

In some examples, a reflector layer, e.g., a layer of optically reflecting material, may be added to the backside of the sensor substrate coupled to the pixelated scintillator, e.g., the pixelated scintillator 20 shown in Figs. 2, 4 and 6. This will be explained hereinafter and in particular with respect to the exemplary radiation detectors shown in Figs. 7 and 8.

In some examples, a reflector layer, e.g., a layer of optically reflecting material, may be added to the bottom of the scintillator elements in the substrate of the pixelated scintillator, e.g., the pixelated scintillator 20 shown in Figs. 2 and 4, or to the backside of the substrate of the pixelated scintillator, e.g., the pixelated scintillator 20 shown in Figs. 2 and 6. This may increase the scintillation light output of the pixelated scintillator.

In some examples, X-ray imaging systems may have a curved pixelated scintillator rather than a flat pixelated scintillator. In this case, the microfabrication of the scintillator elements may still occur on a flat bendable substrate, such as thin glass or plastic foil, which may be subsequently bended slightly in one or two dimensions to meet the desired curvature, e.g., using a mould at elevated temperature, to enable fabrication of a curved radiation detector.

The point spread function of current radiation detectors which are mostly based on continuous scintillator, i.e., non-pixelated scintillator, may be broadened by parallax errors caused by varying absorption depths of incident X-rays in the scintillator layer across the detector. These effects are less pronounced in the centre of the detector (perpendicular incidence of X-rays), but they become stronger towards the edges (oblique incidence of X-rays). To that end, it is a further option for the pixelated scintillator of the present disclosure to be configured to have different thickness of the scintillator along the substrate to realize a uniform scintillator absorption thickness for all pixels across substrate. For example, the at least one scintillator material filled in the plurality of first openings may have a thickness that is reduced from a centre of the substrate towards an edge of the substrate. Such structure may be used to reduce both parallax effects and pixel edge effects. This will be explained in detail hereinafter and in particular with respect to the example shown in Figs. 9(a) - 9(c).

Sensitivity of X-ray detectors based on pixelated scintillators may be limited by an improper match between spectral properties of scintillator and photodiode. With the method as described herein, it is possible to match the emission spectrum of the scintillator material in the scintillator elements with the photodiode sensitivity spectrum in order to improve the DQE. To that end, it is an option for the pixelated scintillator of the present disclosure to be configured to have a composite scintillator material composed of multiple scintillator materials. In some examples, the at least two scintillator materials are deposited in at least one of the first openings consecutively. Alternatively or additionally, at least one scintillator element of the pixelated scintillator comprises a plurality of sub-scintillator elements, and at least two sub-scintillator elements of the at least one scintillator element are filled with different scintillator materials. This will be explained in detail hereinafter and in particular with respect to the example shown in Figs. 10(a) and 10(b).

The pixilated scintillator 20 may be integrated into a radiation detector in various different geometries. For instance, the pixilated scintillator 20 may be integrated into a radiation detector having a front-irradiation geometry, or a back-irradiation geometry, as described above.

Fig. 7 schematically shows an example of a radiation detector 40 fabricated by coupling the pixelated scintillator 20 to a pixelated and photosensitive read-out sensor 30 in the back-irradiation geometry. In this example, the pixelated scintillator 20 is shown having the configuration of Fig. 4. However, it will be appreciated that other configurations, such as the pixelated scintillator 20 shown in Figs. 2 and 6, may also be used in the radiation detector 40. The pixelated and photosensitive read-out sensor 30 comprises a sensor substrate 32 and a photodetector array including a plurality of photosensitive pixels 34 that are in optical communication with the scintillator array 20. In some examples, the photodetector array 34 may be a silicon photomultiplier array, i.e., SiPM, array of photodiodes, for example a Philips Digital Photon Counting, SiPM photodetector array. Alternatively, the photodetector array 34 may include an array of avalanche photodiodes, photomultiplier tubes, position sensitive photodetectors, and so forth. In these examples the photodiodes, photomultipliers, and photodetectors form the photosensitive pixels. The fill factor (i.e., X-ray sensitivity) may be improved by aligning the scintillator elements 18 with photosensitive pixels 34 across the sensor substrate area. Also, empty spaces between scintillator elements18 overlap accurately with dead areas between photosensitive pixels 34. Compared to the front-irradiation geometry, the back-irradiation geometry may improve spatial resolution and sensitivity of a radiation detector. In addition, the back-irradiation geometry may reduce the effective distance between pixelated scintillator and read-out sensor. The effective distance may indicate the average distance of X-ray absorption events to the read-out sensor. The bonding of scintillator to sensor may be realized by e.g. gluing, use of adhesive films, or mechanical clamping. The radiation detector 40 may for example be used in a SPECT, a PET, an X-ray, or a CT imaging system.

Optionally, a reflector layer (not shown), e.g., a layer of optically reflecting material, may be applied to the backside of a sensor substrate in order to reflect some scintillation light emitted from a backlight located below the scintillator substrate back into the photodiodes.

Fig . 8 schematically shows a further example of a radiation detector 40 fabricated by coupling the pixelated scintillator 20 to a read-out sensor 30 in the front-irradiation geometry. Compared to the radiation detection shown in Fig. 7, the pixelated scintillator shown in Fig. 8 is arranged towards X-rays or gamma rays. Therefore, the X-rays or gamma rays first pass through the pixelated scintillator 20 and then hit the read-out sensor 30. The openings that define the scintillator elements may be angulated accordingly.

Optionally, a reflector layer (not shown), e.g., a layer of optically reflecting material, may be applied to the backside of the scintillator substrate. Optionally, a non-transparent material may be applied to the first opening walls (also referred to as scintillator element walls) of the scintillator substrate. Compared to an optically reflecting material, the use of a non-transparent material, e.g., a light-absorbing material, may reduce sensitivity but increase the spatial resolution, which might be interesting for non-medical applications, in which dose is less of importance.

In the following, further radiation detector configurations are illustrated in Figs. 9 to 11. Although these figures may show a pixelated scintillator having a configuration shown in Fig. 4, it will be appreciated that other pixelated scintillators, such as the pixelated scintillator 20 shown in Figs. 2 and 6 may also be implemented in these radiation detector configurations.

Figs. 9(a)-9(c) schematically illustrate how the spatial resolution of a radiation detector in back-irradiation geometry can be improved by optimizing the design of a scintillator coupled to the read-out sensor. As shown in Fig. 9(a), for a non-pixelated scintillator, the resolution reduces from the centre towards the edges of the detector, mainly because of increasing parallax effects caused by obliquely incident X-rays or gamma rays. Fig. 9(b) shows an example of a pixelated non-focused scintillator, in which the at least one scintillator material filled in the plurality of first openings has an identical or similar thickness. In such radiation detector configuration, pixel edge effects originating from scintillator element sidewalls will still reduce spatial resolution towards the edges of the detector. Fig. 9(c) shows an example of a pixelated focused scintillator. In such radiation detector configuration, both parallax effects and pixel edge effects may be reduced, because the scintillator element sidewalls are aligned towards the X-ray focal spot or gamma-ray beam. Moreover, a uniform scintillator absorption depth across the whole detector can be realized by slightly reducing the scintillator thickness from the centre towards the edges. Although Figs. 9(a)-9(c) may illustrate a radiation detector in back-irradiation geometry by way of example, it will be appreciated that a radiation-detection in front-irradiation geometry may also be improved by optimizing the design of a scintillator coupled to the read-out sensor according to the embodiment shown in Fig. 9(c).

Figs. 10(a) and 10(b) show examples in which scintillator elements composed of multiple scintillator materials may be used to improve DQE for specific clinical applications. Generally, this can be achieved by maximizing overlap between scintillator light emission spectrum and photodiode sensitivity spectrum and/or maximizing X-ray or gamma-ray absorption of the scintillator.

The example of Fig. 10(a) shows that three different scintillator layers composed of scintillator materials 18a, 18b, and 18c are consecutively deposited in each scintillator element, which may be realized using a binder jetting device. For example, each photosensitive pixel is bounded to a scintillator element filled with multiple scintillator layers on top of each other. For improved light collection in a back-irradiation geometry, the light emission and radiation absorption of the layer sequence may be chosen such that all layers closest to the read-out sensor are transparent for the light emission of all layers further away from the sensor. Although Fig. 10(a) may show three scintillator layers by way of example, it will be appreciated that in some other examples, the radiation detectors may comprise a different number of scintillator layers, such as two scintillator layers, four scintillator layers, or more scintillator layers.

The example of Fig. 10(b) shows that each scintillator element is composed of three sub-scintillator elements, each of which is filled with a different material. For example, the photosensitive pixel may comprise several smaller photosensitive subpixels, such as subpixels 34a, 34b, and 34c shown in Fig. 10(b), which are coupled to corresponding scintillator sub-scintillator elements filled with different scintillator materials, such as the scintillator material 18a, 18b, 18c shown in Fig. 10(b). Radiation absorption characteristics of scintillator sub-scintillator elements might be chosen to achieve maximal differentiation between object materials in spectral imaging. Although Fig. 10(b) may show three subpixels by way of example, it will be appreciated that in some other examples, each photosensitive pixel may comprise a different number of subpixels, such as two subpixels, four subpixels, or more subpixels.

Although Figs. 10(a) and 10(b) may illustrate a radiation detector in back-irradiation geometry by way of example, it will be appreciated that a radiation-detection in front-irradiation geometry may also be improved by including scintillator elements composed of multiple scintillator materials according to the examples shown in Figs. 10(a) and 10(b).

Figs. 11(a)-11(c) illustrate that pixelated focused scintillators are advantageous for multi-layer detectors to minimize cross contamination of signals between different detector layers. The accurate stacking and alignment of corresponding focused scintillator elements, which may be enabled by their accurate positioning on glass substrates using femto-laser micro-structuring, may ensure that cross contamination of signals between detector layers is reduced.

Fig. 11(a) shows a dual-layer radiation detector based on two sensors, and Fig. 11(b) shows a triple-layer radiation detector based on three sensors. Both radiation detectors are in the back-irradiation geometry with standard stacking of sensors. Fig. 11(c) shows an alternative triple-layer radiation detector based on two sensors. In this case the bottom sensor is photosensitive on both sides (a so-called "bidirectional" sensor), i.e., pixels are designed to capture scintillation light emitted from scintillator elements at the top and at the bottom. Although Figs. 11(a) - 11(c) may illustrate a radiation detector in back-irradiation geometry by way of example, it will be appreciated that a radiation-detection in front-irradiation geometry may also be improved with the exemplary configurations shown in Figs. 11(a) - 11(c). In some examples, such as the example shown in Fig. 11(c), the radiation detector may be configured to have both front-irradiation geometry and back-irradiation geometry.

Fig. 12 illustrates an example of a system 200 for manufacturing a pixelated scintillator. The system 200 comprises an optical laser system 210, a chemical etching system 220, a material depositing system 230, and a control system 240.

The control system 240 may comprise one or more controllers. Examples of controller components that may be employed in various embodiments of the present disclosure include, but are not limited to, conventional microprocessors, application specific integrated circuits (ASICs), and field-programmable gate arrays (FPGAs). The control system 240 is configured to control the optical laser system 210 to irradiate first laser radiation to a plurality of first regions of a substrate according to a predefined geometry of the pixelated scintillator to cause a structural modification of a material in the plurality of first regions of the substrate such that wet chemical etching occurs at a higher rate at the plurality of irradiated first regions than at a non-irradiated region.

The control system 240 is configured to control the chemical etching system 220 to perform wet chemical etching to form a plurality of first openings having the predefined geometry of the pixelated scintillator to form scintillator elements of the pixelated scintillator.

The control system 240 is configured to control the material depositing system 230 to fill the plurality of first openings with at least one scintillator material to form the pixelated scintillator.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A method of manufacturing a pixelated scintillator, the method comprising:
- irradiating (S110), with first laser radiation, a plurality of first regions of a substrate according to a predefined geometry of the pixelated scintillator to cause a structural modification of a material in the plurality of first regions of the substrate such that wet chemical etching occurs at a higher rate at the plurality of irradiated first regions than at a non-irradiated region;
- performing (S120) wet chemical etching to form a plurality of first openings having the predefined geometry of the pixelated scintillator; and
- filling (S130) the plurality of first openings with at least one scintillator material to form the pixelated scintillator.

2. The method according to claim 1,
wherein prior to step of filling (S130) the plurality of first openings with at least one scintillator material to form the pixelated scintillator, the method further comprises:
- coating (S122) at least part of an interior surface of at least one first opening with an optically reflecting material or a non-transparent material.

3. The method according to claim 1 or claim 2,
wherein after the step of filling (S130) the plurality of first openings with at least one scintillator material to form the pixelated scintillator, the method further comprises:
- coating at least one filled first opening with an optically reflecting material or a non-transparent material.

4. The method according to any one of the preceding claims, further comprising:
- irradiating (S102), with second laser radiation, one or more second regions of the substrate,
- performing (S 104) wet etching to form one or more second openings, and
- filling (S106) the one or more second openings with an optically reflecting material or a non-transparent material, wherein the one or more second regions are different from the plurality of first regions, and at least one second region is arranged between two adjacent first regions.

5. The method according to any one of the preceding claims, further comprising:
- bending the substrate in one or two dimensions to shape the substrate according to a desired curvature.

6. The method according to any one of the preceding claims,
wherein at least one of the first laser radiation and the second laser radiation is produced by a focused pulsed femtosecond laser.

7. The method according to any one of the preceding claims,
wherein the plurality of first openings are angulated towards a common focal point.

8. The method according to any one of the preceding claims,
wherein the at least one scintillator material filled in the plurality of first openings has a relatively greater thickness that is in a centre of the substrate than at an edge of the substrate.

9. The method according to any one of the preceding claims,
wherein at least two scintillator materials (18a, 18b, 18c) with different scintillator light emission spectra are filled in the plurality of first openings.

10. The method according to claim 9,
wherein the at least two scintillator materials are deposited in at least one of the first openings consecutively; and/or
wherein the pixelated scintillator comprises a plurality of scintillator elements, and wherein at least one scintillator element of the pixelated scintillator comprises a plurality of sub-scintillator elements, and at least two sub-scintillator elements of the at least one scintillator element are filled with different scintillator materials.

11. The method according to any one of the preceding claims,
wherein the substrate comprises a glass material or a plastic material.

12. A pixelated scintillator (20) obtainable by a method according to any one of the preceding claims.

13. A radiation detector (40), comprising:
- a pixelated scintillator (20) according to claim 12; and
- a pixelated and photosensitive read-out sensor (30).

14. The radiation detector according to claim 13,
wherein the radiation detector is a multi-layer detector comprising a plurality of stacked pixelated scintillators.

15. The radiation detector according to claim 13 or claim 14,
wherein the pixelated scintillator and the pixelated and photosensitive read-out sensor are configured and arranged to form the radiation detector in a back-irradiation geometry or in a front-irradiation geometry.
